# EUROPEAN PATENT SPECIFICATION

(11) **EP 1 477 136 B1**
(45) Date of publication and mention of the grant of the patent: **24.10.2007**
(21) Application number: 04017595.2
(22) Date of filing: 24.09.1999
(51) Int. Cl.: A61F 2/06

(54) **Longitudinally flexible expandable stent**
Längsflexibler und expandierbarer Stent
Stent extensible et longitudinalement flexible

(30) Priority: 20.11.1998 US 197278
(43) Date of publication of application: 17.11.2004
(62) Divisional of application: 99952949.8
(73) Proprietor: Boston Scientific Limited, St. Michael, Barbados, West Indies (BB)
(72) Inventor: Brown, Brian J., Hanover MN 55341 (US); Davis, Michael, Shorewood MN 55311 (US); Friesen, David, Brooklyn Park MN 55444 (US); Ley, Timothy J., Shoreview MN 55126-1905 (US); Skubitz, Sean P., Shoreview MN 55126 (US)
(74) Representative: Hauck Patent- und Rechtsanwälte

(56) References cited:
- EP-A- 0 876 806
- WO-A-96/21404
- WO-A-96/26689

## Description

### Field of the Invention

This invention relates to an endoprosthesis device for implantation within a body vessel, typically a blood vessel. More specifically, it relates to a tubular expandable stent of improved longitudinal flexibility.

### Background of the Invention

Stents are placed or implanted within a blood vessel for treating stenoses, strictures or aneurysms therein. They are implanted to reinforce collapsing, partially occluded, weakened, or dilated sections of a blood vessel. They have also been implanted in the urinary tract and in bile ducts.

Typically, a stent will have an unexpanded (closed) diameter for placement and an expanded (opened) diameter after placement in the vessel or the duct. Some stents are self expanding and some are expanded mechanically with radial outward force from within the stent, as by inflation of a balloon.

An example of the latter type is shown in U.S. Patent No. 4,733,665 which discloses a number of stent configurations for implantation with the aid of a catheter. The catheter includes an arrangement wherein a balloon inside the stent is inflated to expand the stent by plastically deforming it, after positioning it within a blood vessel.

A type of self-expanding stent is described in U.S. Patent No. 4,503,569 which discloses a shape memory stent which expands to an implanted configuration with a change in temperature. Other types of self-expanding stents not made of shape memory material are also known.

This invention is directed to stents of all these types when configured so as to be longitudinally flexible as described in detail hereinbelow. Flexibility is a desirable feature in a stent so as to conform to bends in a vessel. Such stents are known in the prior art. Examples are shown in U.S. Patent No. 4,856,516 to Hillstead; U.S. Patent No. 5,104,404 to Wolff; U.S. Patent No. 4,994,071 to MacGregor; U.S. Patent No. 5,102,417 to Palmaz; U.S. Patent No. 5,195,984 to Schatz; U.S. Patent No. 5,135,536 to Hillstead; U.S. Patent 5,354,309 to Shepp-Pesch et al.; EPO Patent Application 0 540 290 A2 to Lau; EPO Patent Application No. 0 364 787 B1 to Schatz, and PCT Application WO 94/17754.

Generally speaking, these kinds of stents are articulated and are usually formed of a plurality of aligned, expandable, relatively inflexible, circular segments which are interconnected by flexible elements to form a generally tubular body which is capable of a degree of articulation or bending. Unfortunately, a problem with such stents is that binding, overlapping or interference can occur between adjacent segments on the inside of a bend due to the segments moving toward each other and into contact or on the outside of a bend the segments can move away from each other, leaving large gaps. This can lead to improper vessel support, vessel trauma, flow disturbance, kinking, balloon burst during expansion, and difficult recross for devices to be installed through already implanted devices and to unsupported regions of vessel.

A diamond configuration with diagonal connections between each and every diamond of each segment is also known but such closed configurations lack flexibility.

WO 96/21404 discloses a kink-resistent stent-graft. The stent-graft may comprise torsion members on the stent having different amplitudes. The peaks of the high amplitude torsion members may be lined up "out of phase" or "peak to peak" with short amplitude or high amplitude torsion members in the adjacent turn of a helix or may be positioned "in phase".

WO 96/26689 discloses a longitudinally flexible expandable stent. The stent comprises a plurality of cylindrical shaped segments interconnected by substantially parallel struts. The cylindrical shaped segments are formed in an undulating pattern of interconnected substantially parallel struts, having the same length.

EP 0 876 806 A1 discloses a stent having cylindrical segments interconnected by connectors extending under an angle with respect to the longitudinal axis of the stent. The cylindrical segments are formed of struts having essentially the same length.

US 5,725,572 discloses a radio-opaque stent. The stent comprises cylindrical elements having M-shaped elements and U-shaped elements alternating along the cylindrical element. M-shaped and U-shaped elements have a different amplitude in the cylindrical segment.

It is an object of this invention to provide a longitudinally flexible stent of open configuration having improved flexibility (radially and longitudinally) in the stent body segments thereof rather than in flexible joints between the segments.

The problem is solved by the stent of claim 1.

### Brief Description of the Figures

- Figure 1: shows a band-like element used for stents.
- Figure 1b: shows a schematic of a peak region which contains a double peak and a trough region containing a double trough.
- Figure 2: shows a flat view of a stent configuration.
- Figure 3: shows the pattern of Figure 2 in a tubular stent.
- Figure 4a: shows a flat view of a stent configuration.
- Figure 4b: shows a flat view of a stent configuration.
- Figure 5a: shows a flat view of a stent configuration.
- Figure 5b: shows a flat view of a stent configuration.
- Figure 6: shows a flat view of a stent configuration.
- Figure 7: shows a flat view of a stent configuration.
- Figure 8: shows a flat view of a stent configuration.
- Figure 9: shows a flat view of a stent configuration according to the invention.
- Figure 10: shows a flat view of a stent configuration.
- Figure 11: shows a flat view of a stent configuration.
- Figure 12: shows a flat view of a stent configuration.
- Figure 13: shows the pattern of Figure 12 in a tubular stent.
- Figure 14: shows an expanded stent of the configuration shown in Figure 12.
- Figure 15: shows a flat view of an alternate stent configuration.

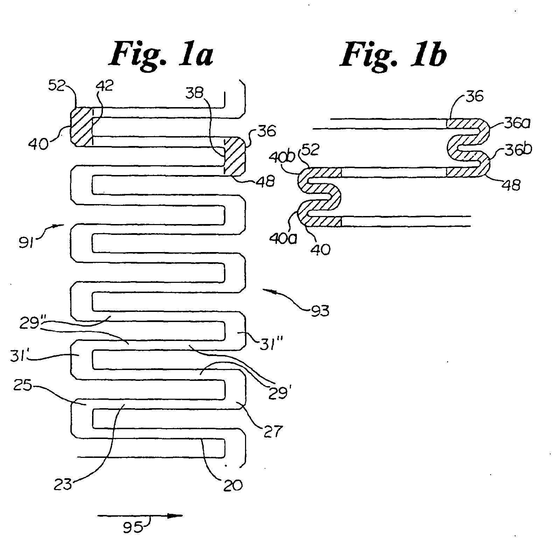

Figs. 1 - 8 and 10-15 show stent configurations not according to the invention.

### Detailed Description of the Invention

While this invention may be embodied in different forms, there are described in detail herein specific preferred embodiments of the invention.

For the sake of consistency, the terms 'peak' and 'trough' shall be defined with respect to the proximal and distal ends of the stent. Each of the stents has a proximal end 91 and a distal end 93 and a longitudinal axis 95, as seen in Fig. 1a. Peaks 36 are generally concave relative to the proximal end of the stent and generally convex relative to the distal end of the stent. Troughs 40, on the other hand, are generally convex relative to the proximal end of the stent and generally concave relative to the distal end of the stent. Notwithstanding this definition, the term peak is also intended to extend to regions 48 that are generally peak-like which may, nevertheless, contain trough-like regions within the peak-like region as seen in Fig. 1b. Similarly the term trough is also intended to extend to regions 52 that are generally trough-like which may, nevertheless, contain peak-like regions within the trough-like region as seen in Fig. 1b.

Corresponding to each peak 36 is an inner diameter peak 38 where the inner diameter of the band-like element reaches its peak. The set of points on a given band-like element which are distal to inner diameter peak 38 is denoted peak region 48. Similarly, corresponding to each trough 40 is an inner diameter trough 42 where the inner diameter of the band-like element reaches its trough. The set of points on a given band-like element which are proximal to inner diameter trough 42 is denoted trough region 52. For the sake of clarity, unless otherwise indicated, analogous portions of stents will be similarly labeled, using three digit reference numerals to distinguish among the various embodiments shown.

Also included within this definition of peak regions and trough regions are peak regions which are comprised of multiple peaks as well as trough regions which are comprised of multiple troughs such as those shown schematically in Fig 1b. Peak 36 is seen to consist of two sub-peaks 36a,b and trough 40 is similarly seen to consist of two sub-troughs 40a,b. In the case of peaks containing sub-peak and troughs containing sub-troughs, the peak region 48 includes all of the points along the band-like element between the sub-peaks that make up the peak and similarly, the trough region 52 includes all of the points along the band-like element between the sub-troughs that make up the trough.

The inventive stents may incorporate one or more bands of a chosen wavelength. The inventive stents include one or more small amplitude, short wavelength bands to provide for flexibility and one or more large amplitude, long wavelength bands to give side branch access or to provide for sections of alternative strengths such as soft and/or stiff sections.

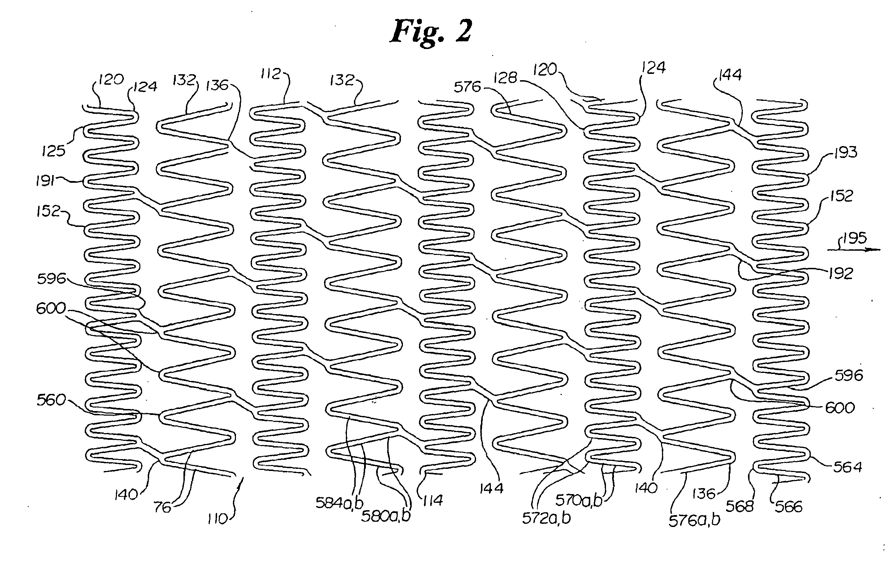

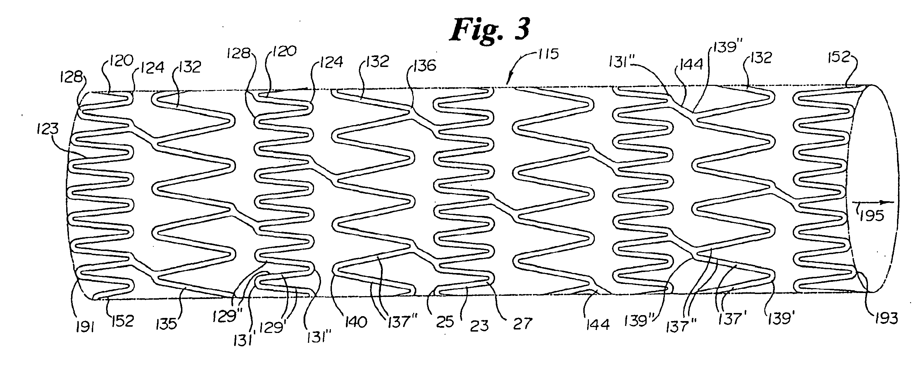

Turning to the Figures, Figure 2 shows a flat view of a stent configuration and Figure 3 shows the stent of Figure 2 in tubular form. That is, the stent is shown for clarity in Figure 2 in the flat and may be made from a flat pattern 110 (Figure 2) which is formed into a tubular shape by rolling the pattern so as to bring edges 112 and 114 together (Figure 2). The edges may then joined as by welding or the like to provide a cylindrical configuration such as that shown generally at 115 in Figure 3.

A more preferred method of manufacture begins with a thin walled tube which is then laser cut to provide the desired configuration. It may also be chemically etched or EDM'd (electrical discharge machined) to form an appropriate configuration.

The configuration can be seen in these Figures to be made up of one or more spaced first band-like elements 120. First band-like elements have a generally serpentine configuration to provide continuous waves to the first band-like elements. The waves are characterized by a plurality of peaks 124 and troughs 128 taking a generally longitudinal direction along the cylinder such that the waves in first band-like elements 120 open as the stent is expanded from an unexpanded state having a first diameter to an expanded state having a second diameter.

The stent further comprises a plurality of spaced second band-like elements 132 having a generally serpentine configuration to provide continuous waves to the second band-like elements. The waves are characterized by a plurality of peaks 136 and troughs 140 taking a generally longitudinal direction along the cylinder such that the waves in the second band-like elements open as the stent is expanded from an unexpanded state having a first diameter to an expanded state having a second diameter. First and second band-like elements are characterized by respective wavelengths and amplitudes with the wavelength and amplitude of the second band-like elements exceeding the wavelength and amplitude of the first band-like elements.

Adjacent first band-like elements 120 and second band-like elements 132 are interconnected via a plurality of interconnecting elements 144. The ends of interconnecting element are circumferentially offset from each other.

In a stent as shown in Figs. 2 and 3, first band-like elements 120 and second band-like elements 132 alternate over the length of the stent. Optionally, as shown in Figs. 2 and 3, each end 152 of the stent may terminate in a first band-like element. One example, however, contemplates each end terminating in a second band-like element, or further, one end terminating in a first band-like element and the other end terminating in a second band-like element.

While a minimum of one connecting element is required to join adjacent band-like elements, two or more interconnecting elements are preferred. In one stent, as shown in Figs. 2 and 3, adjacent first and second band-like elements 120 and 132 are connected with three interconnecting elements 144. Further, in one stent, adjacent interconnecting elements 144 extending from peaks 136 on a first band-like element 120 are spaced five peaks apart on the first band-like element while adjacent interconnecting elements 144 extending from troughs 140 on a second band-like element 132 are spaced three troughs apart on the second band-like element.

It is a further feature that peaks 124 on first band-like elements 120 are circumferentially displaced on the periphery of the stent from troughs 140 on adjacent second band-like elements 132. It is desirable that peaks and troughs be displaced in the expanded state of the stent to minimize the possibility of pinching or overlap between adjacent band-like elements.

Although the stent of Fig. 2 is comprised of two different wavelength band-like elements, the invention contemplates stents with a plurality of different wavelength band-like elements. As such, other stents may have three, four or more different wavelength band-like elements.

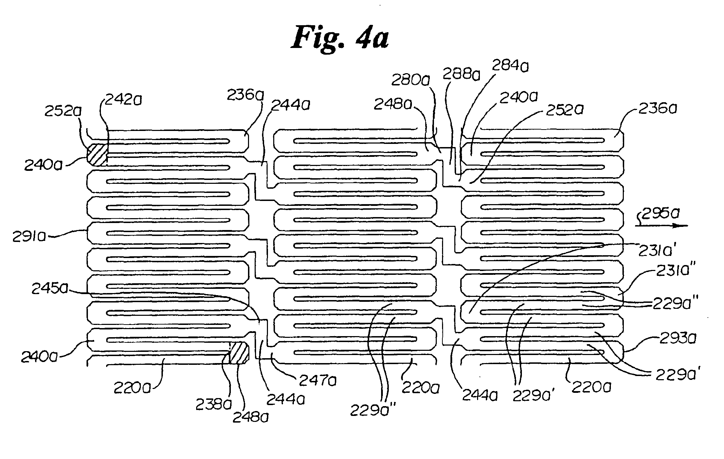

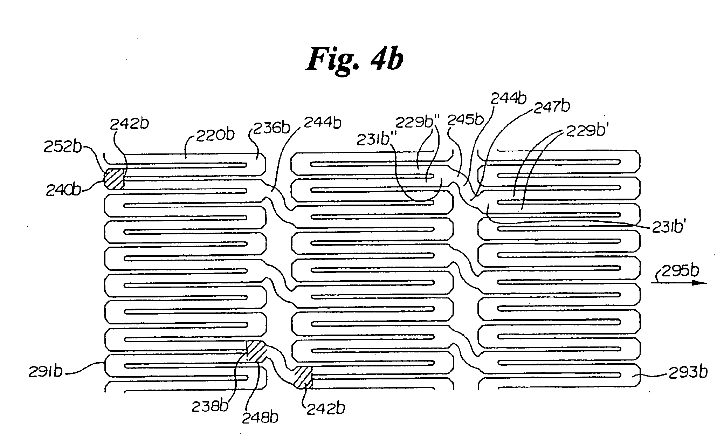

In another example, the stent is comprised of band-like elements of a single wavelength, interconnected by interconnecting elements. Turning to Figs. 4a and 4b, band-like elements 220a, b are interconnected by interconnecting elements 244a,b. Adjacent band-like elements 220a, b are 180 DEG out of phase with one another. In the compressed state, the band-like elements consist of a plurality of peaks 236a,b and troughs 240a,b. Peak region 248a,b and trough region 252a,b have been shaded in one instance for illustrative purposes.

In the example shown in Fig. 4a, each interconnecting element 244a extends between a peak region 248a and a trough region 252a. Rectilinear interconnecting elements 244a consist of a first shank 280a, a second shank 284a and a link 288a disposed in-between the first and second shanks 280a and 284a. First shank 280a extends in a longitudinal direction from peak region 248a and is substantially perpendicular to link 288a. Second shank 284a extends in a longitudinal direction from trough region 252a and is perpendicular to link 288a.

In the example shown in Fig. 4b, the stent differs from the example of Fig. 4a in that interconnecting element 244b extending between a peak region 248b and a trough region 252b is curvilinear rather than rectilinear.

In both figures 4a and 4b, the interconnecting elements are seen to emanate from the middle of the peak and trough regions.

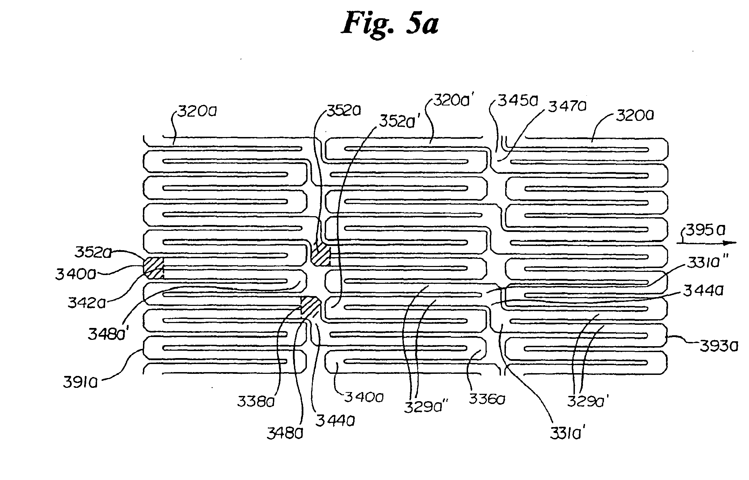

In another example, as shown in Fig. 5a, the stent is comprised of band-like elements 320a of a single wavelength, interconnected by interconnecting elements 344a. Adjacent band-like elements 320a are 180 DEG out of phase with one another. The band-like elements consist of a plurality of peaks 336a and troughs 340a. Interconnecting elements 344a extend between a peak region 348a and a trough region 352a. The peak regions 348a and trough regions 352a from which interconnecting elements 344a emanate on a given band-like element 320a are seen to extend longitudinally beyond adjacent peak regions 348a' and trough regions 352a' from which no interconnecting elements extend. The extension is such that at least a portion of peak regions 348a overlap longitudinally along the stent with at least a portion of trough region 352a on an adjacent band-like element 320a'. Of course, the overlap is limited to the longitudinal direction and not to the circumferential direction.

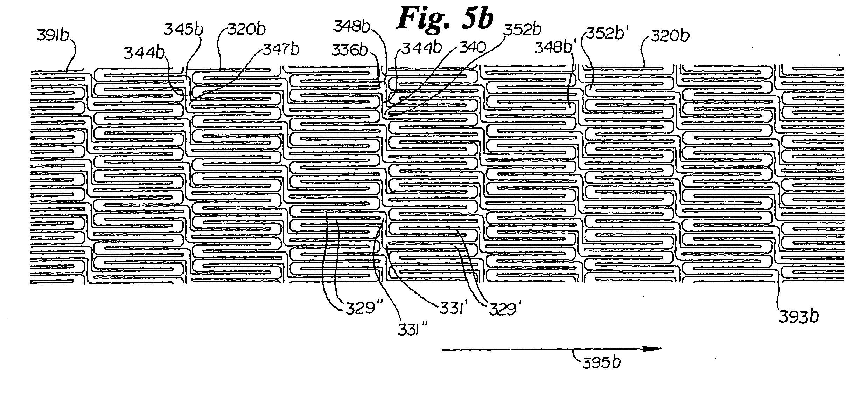

In another example, as shown in Fig. 5b, interconnecting elements 344b extend between peak region 348b and a second closest trough region 352b on an adjacent band-like element. Interconnecting elements 344b are seen to be perpendicular to the longitudinal axis. As in the stent of Fig. 5a, peak regions 348b from which interconnecting elements 344b extend and trough regions 352b from which interconnecting elements 344b extend may extend beyond adjacent peak regions 348b' and trough regions 352b' from which no interconnecting elements 344b emanates.

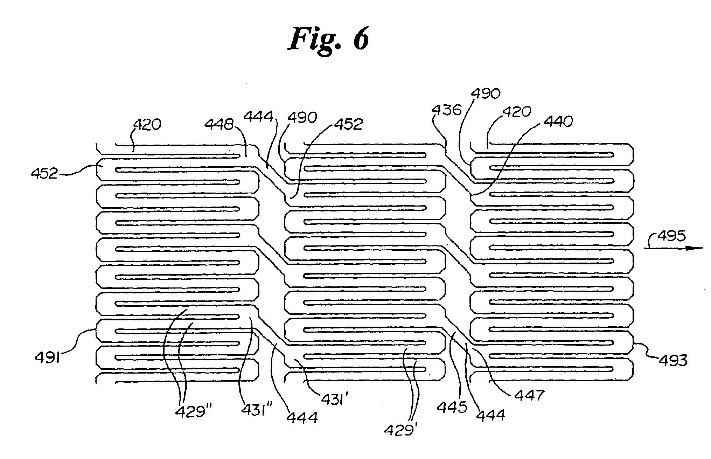

In another example, as shown in Fig. 6, adjacent band-like elements 420 are in phase with each other. As in previous Figs, band-like elements 420 are of a single wavelength, interconnected by interconnecting elements 444. The band-like elements consist of a plurality of peaks 436 and troughs 440. Interconnecting elements 444 extend at an oblique angle relative to the longitudinal axis of the stent between a peak region 448 and a trough region 452. As such, ends of interconnecting elements 444 are circumferentially offset relative to each other. The exact angle will, of course, depend on the region from which the interconnecting elements extend, as well as on whether interconnecting elements interconnect nearest peaks and troughs, next nearest peaks and troughs or peaks and troughs that are further separated.

In Figures 5a, 5b and 6, the interconnecting elements are seen to emanate from the sides of the peak and trough regions.

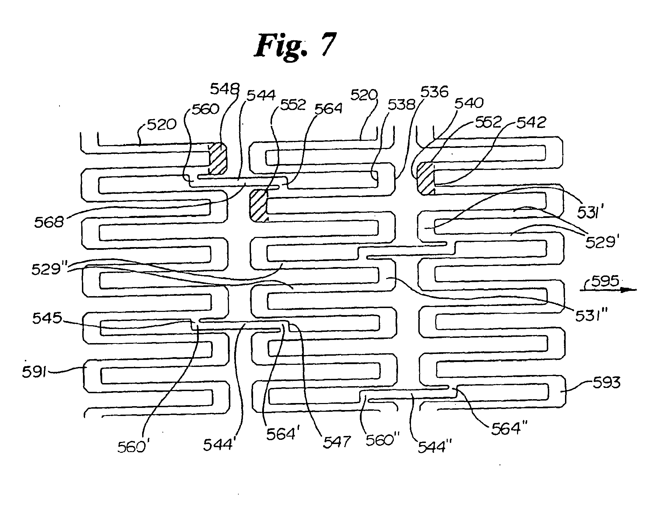

Although for the examples of Figs. 1-6, the interconnecting elements extend from peak regions on band-like elements to trough regions on adjacent band-like elements, another example further contemplates interconnecting elements extending from a position between a peak region and an adjacent trough region on a band-like element to a position intermediate a trough region and a peak region on an adjacent second band-like element as in Fig. 7.

In the example of Fig. 7, interconnecting elements are seen to extend from a region between the peak region and the trough region on a band-like element. The stent is formed of adjacent band-like elements 520 which are 180 DEG degrees out of phase with one another. Interconnecting elements 544 extend from a region intermediate a peak region 548 and a trough region 552 on a band-like element to a region intermediate a peak region 548 and a trough region 552 on an adjacent band-like element. Interconnecting elements 544 consist of a first shank 560, a second shank 564, and an intermediate member 568 disposed in-between first and second shanks 560 and 564. First shank 560 and second shank 564 are substantially perpendicular to intermediate member 568 which extends in the longitudinal direction. Although not depicted, the region from which interconnecting elements 544 emanate may be midway between peaks and troughs.

The example of Fig. 7 also differs from the examples of Figs. 2-6 in the orientation of the interconnecting elements. Whereas the interconnecting elements in Figs. 2-6 are all similarly oriented, in the example of Fig. 7, the orientation of interconnecting elements alternates between adjacent pairs of adjacent band-like elements. Specifically, second shanks 564' of interconnecting elements 544' are seen to be displaced in a clockwise circumferential direction along the stent relative to first shanks 560', and seconds shank 564" of interconnecting elements 544" are seen to be displaced in a counterclockwise circumferential direction along the stent relative to while first shank 560".

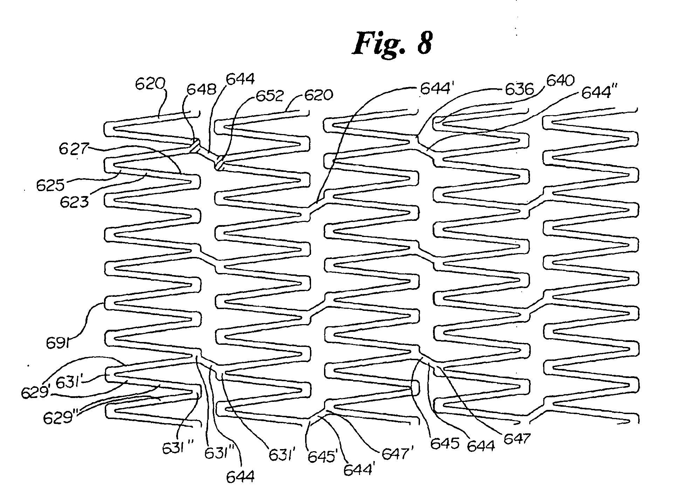

This feature is also seen in the example of Fig. 8 in which adjacent in-phase band-like elements 620 are interconnected by interconnecting elements 644. Interconnecting elements 644 extend at an oblique angle relative to the longitudinal axis of the stent between a peak region 648 and a trough region 652. As in Fig. 7, the orientation of interconnecting elements alternates between adjacent pairs of adjacent band-like elements. Specifically, the distal ends of interconnecting elements 644' are seen to be oriented in a counterclockwise circumferential direction along the stent relative to the proximal end of the interconnecting elements while the distal ends of interconnecting elements 644" are seen to be displaced in a clockwise circumferential direction along the stent relative to the proximal ends.

Although in the example of Figs 2-8, adjacent bands are connected by five interconnecting elements, additional or fewer interconnecting elements may be used. Further, while interconnecting elements are shown spaced three peaks apart and three troughs apart, other separations are contemplated as well.

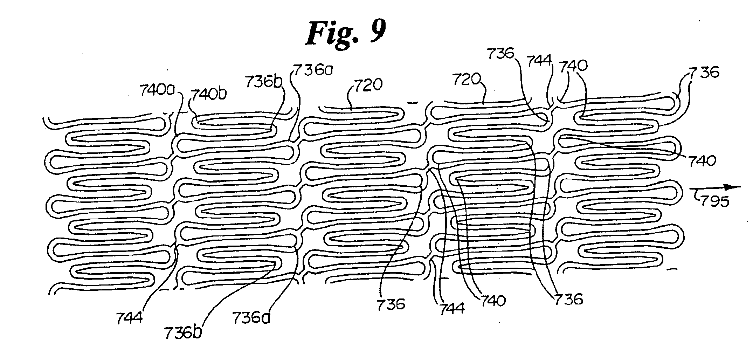

In the embodiment of Figure 9, each band-like element 720 is seen to comprise peaks 736 of more than one amplitude and troughs 740 of more than one amplitude. Large amplitude peaks 736a and small amplitude peaks 736b alternate as do large amplitude troughs 740a and small amplitude troughs 740b. As in the previous examples, the interconnecting elements are oriented at an oblique angle relative to the longitudinal axis 795 of the stent. More generally, the invention is directed at stents comprising band-like elements whose amplitude varies along the band-like element.

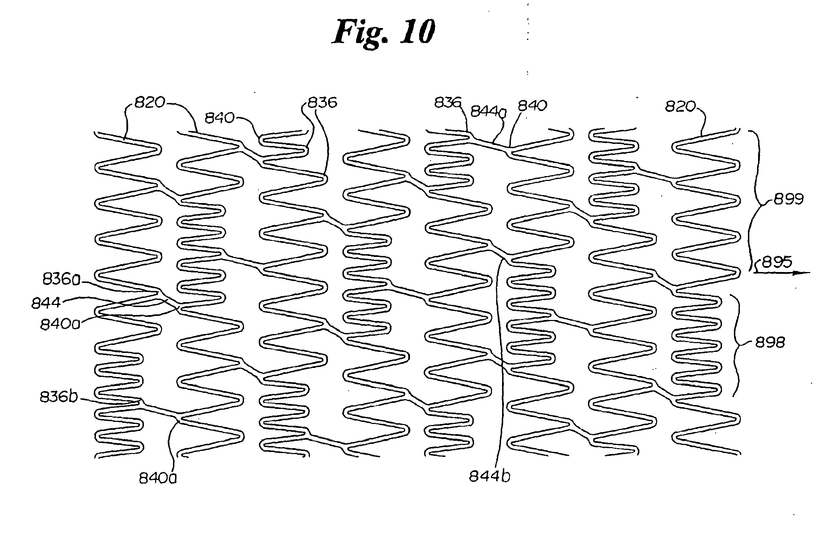

In another example, as shown in Fig. 10, each band-like element 820 is seen to comprise peaks 836 of more than one amplitude and troughs 840 of more than one amplitude, however, peaks of the same amplitude are grouped together within a band-like element as are troughs of the same amplitude. It is further noted that in the example of Figure 10, the location of a group of peaks of given amplitude in a band-like element varies circumferentially along the length of the stent. Interconnecting elements 844 connect peaks 836 and troughs 840 in adjacent band-like elements 820. Where several peaks of different amplitudes are present in a band-like element, the invention as claimed further contemplates the possibility of interconnecting elements extending from the large peaks 836a to large troughs 840a as in Fig. 9. There is the possibility of interconnecting elements extending from large peaks to small troughs or from small peaks 836b to large troughs 840a as in the example of Fig. 10. Further, the interconnecting elements between any two adjacent band-like elements may be of different lengths from one another as seen in Fig. 10 and commence at different longitudinal positions within a band-like element and terminate at different longitudinal positions within a band-like element. Interconnecting element 844a is seen to be longer than interconnecting element 844b. As in the previous examples, the interconnecting elements are oriented at an oblique angle relative to the longitudinal axis 895 of the stent. In the example of Fig. 10, interconnecting element 844a is seen to be oriented at a smaller oblique angle relative to the longitudinal axis of the stent than interconnecting element 844b. As is apparent from Fig. 10, this example is directed to stents comprised of band-like elements whose wavelength varies along a given band-like element. Region 898 and region 899 of band-like element are characterized by different wavelengths.

It is also noted that in the example of Fig. 10, all of the troughs 840a,b in a given band-like element 820 are aligned longitudinally along the stent and differ only in their circumferential position along the stent.

It is further noted in the example of Fig. 10, the stent comprises a first group of interconnecting elements 844a and a second group of interconnecting elements 844b. The interconnecting elements of the first group are all parallel to one another and disposed at a different oblique angle relative to the longitudinal axis than the members of the second group which are all parallel to one another. As such, the example contemplates stents having several different groups of obliquely disposed interconnecting elements where the oblique angle differs from group to group.

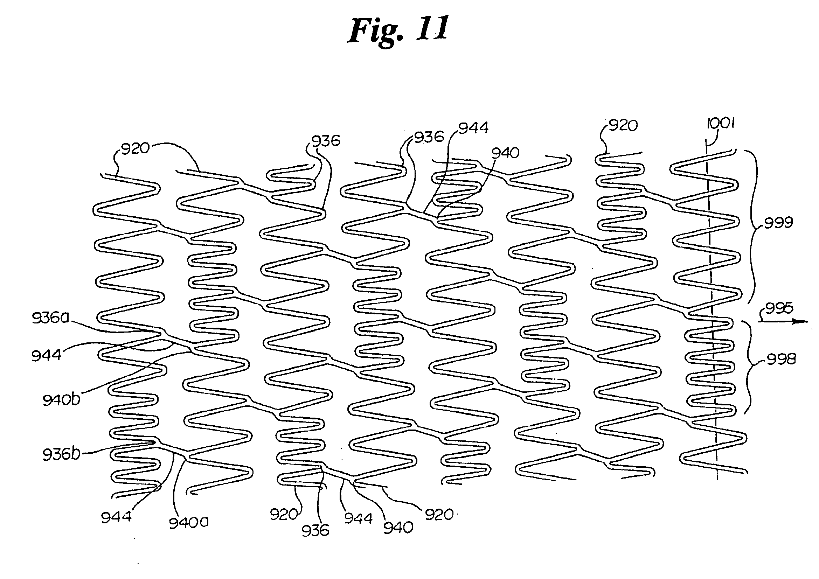

In another example, as shown in Fig. 11, each band-like element 920 is seen to comprise peaks 936a,b of different amplitudes and troughs 940 of different amplitudes, however, peaks of the same amplitude are grouped together within a band-like element as are troughs of the same amplitude. It is further noted that in the example of Figure 11 the location of groups of peaks of given amplitude in a band-like element varies circumferentially along the length of the stent. Interconnecting elements 944 connect large amplitude peaks 93 6a and small amplitude troughs 940b in adjacent band-like elements 920. Similarly, interconnecting elements 944 also connect small amplitude peaks 936b and large amplitude troughs 940a.

The invention also contemplates stents similar to the example shown in Fig. 11 in which interconnecting elements extend from large peaks 936a to large troughs 940a, as in Fig. 9. Similarly, interconnecting elements may extend from small peaks 936b to small troughs 940b.

Further, the interconnecting elements between any two adjacent band-like elements may be of different lengths from one another and disposed at different oblique angles.

As is apparent from Fig. 11, this example shows a stent comprised of band-like elements whose wavelength varies along a given band-like element. Region 998 and region 999 of band-like element 920 are characterized by different wavelengths.

It is also noted that in the example of Fig. 11 the large amplitude portions 999 of band-like element 920 are symmetrically disposed about the center 1001 of the band-like element as are the small amplitude portions 998. The center 1001 of the band-like element is defined as a ring that runs along a path that is midway between the large peaks 936a and large troughs 940a of the band-like element. This feature may also be seen in the embodiment of Fig. 9.

Some examples are also directed to a tubular, flexible, expandable stent having a longitudinal axis, comprising one or more cylindrical shaped first segments. Cylindrical shaped first segments 20 as seen in Fig. 1, have first struts 23 having first 25 and second 27 ends. First segments 20 are defined by a member formed in an undulating pattern of interconnected paired first struts 23, in which adjacent pairs of first struts 29' and 29" in a given first segment 20 are interconnected at opposite ends 31' and 31", respectively. Adjacent segments are interconnected.

Some examples of the stent may be seen more clearly in Figs. 2-8. As shown, the stent of Fig. 3, in addition to comprising first segments 120 which are defined by an undulating pattern of interconnected paired first struts 123 in which adjacent pairs of first struts 129' and 129" in a given first segment 120 are interconnected at opposite ends 131' and 131 ", respectively, the stent further comprises one or more cylindrical shaped second segments 132, each second segment being defined by a member formed in an undulating pattern of interconnected paired second struts 135 and in which adjacent pairs of second struts 137' and 137" in a given second segment 132 are interconnected at opposite ends 139' and 139", respectively. First struts 123 are shorter than second struts 135. First segments 120 are formed of a number of first struts 123 and second segments 132 formed of a number of second struts 135, the number of first struts in a first segment exceeding the number of second struts in a second segment. First and second segments 120 and 132 are aligned on a common longitudinal axis 195 to define a generally tubular stent body, shown generally at 115. First and second segments 120 and 132 alternate along the stent body. Adjacent first and second segments 120 and 132 are connected by a plurality of interconnecting elements 144. Each interconnecting element 144 extends from an end 131" of paired first struts on a first segment 120 to an end 139" of paired second struts on an adjacent second segment 132. The ends of interconnecting elements 144 are circumferentially offset relative to each other.

Desirably, upon expansion of stent 115, paired struts 129" and 137" of adjacent segments 120 and 132 are displaced relative to each other about the periphery of the stent body to accommodate longitudinal flexing of the stent within the segments and without interference between adjacent segments.

In the examples as shown in Figs. 4a, b, cylindrical shaped segments 220a,b are formed of interconnected struts 223a,b having first 225 and second 227 ends. Adjacent pairs of struts 229a,b' and 229a,b" in a given segment 220a,b are interconnected at opposite ends 231a,b' and 231a,b", respectively. Adjacent segments are connected by a plurality of interconnecting elements 244a,b. Each interconnecting element 244a,b extends from an end of paired struts 231a,b" on a segment to an end of paired struts 231a,b' on an adjacent segment. First end 245a,b and second end 247a,b of interconnecting elements 244a,b are seen to be circumferentially displaced along the stent.

Similar structure, denoted by similar reference numerals may be found in the stents of Figs. 5a,b, and 6-8.

In particular, in the example as shown in Fig. 8, cylindrical shaped segments 620 are formed of interconnected struts 623, having first 625 and second 627 ends. Segments 620 are defined by a member formed in an undulating pattern of interconnected paired struts 623 in which adjacent pairs of struts 629' and 629" in a given segment 620 are interconnected at opposite ends 631' and 631", respectively. Segments 620 are aligned on a common longitudinal axis 695 to define a generally tubular stent body. Adjacent segments are connected by a plurality of interconnecting elements 644 (and 644') having first 645 (645') and second 647 (647') ends, each interconnecting element 644 (644') extending from an end of paired struts 631" on a segment to an end of paired struts 631' on an adjacent segment. First end 645 (645') and second end 647 (647") are seen to be circumferentially displaced along the stent.

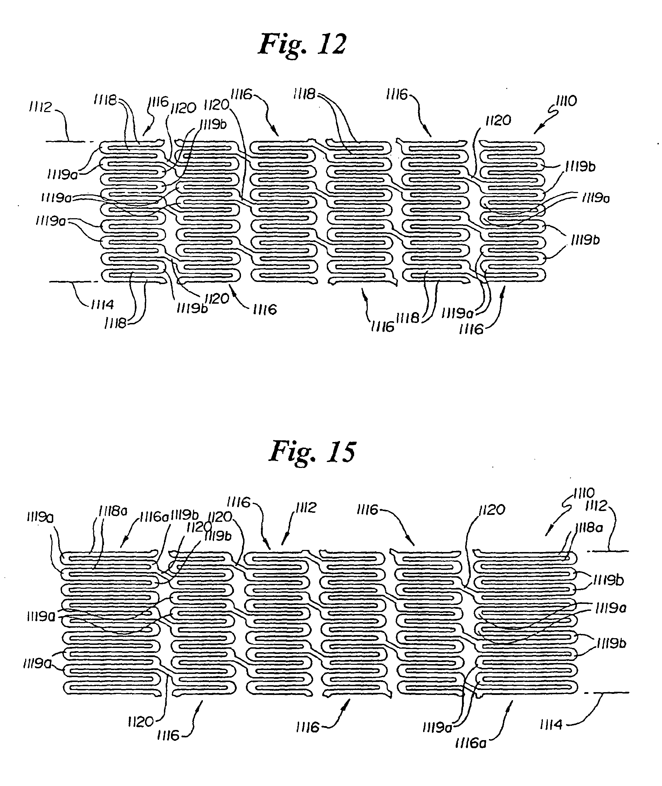

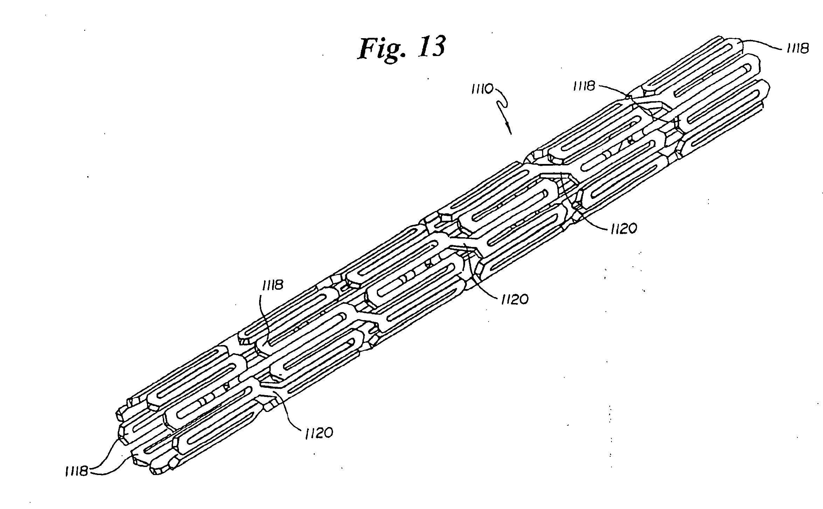

Additional examples of the stents are shown in Figs 12-15. Figure 12 and Figure 13 show a fragmentary flat view of an unexpanded stent configuration and the actual tubular stent (unexpanded), respectively. That is, the stent is shown for clarity in Figure 12 in the flat and may be made from a flat pattern 1110 (Figure 12) which is formed into a tubular shape by rolling the pattern so as to bring edges 1112 and 1114 together (Figure 12). The edges may then joined as by welding or the like to provide a configuration such as that shown in Figure 13.

The configuration can be seen in these Figures to be made up of a plurality of adjacent segments generally indicated at 1116, each of which is formed in an undulating flexible pattern of substantially parallel struts 1118. Pairs of struts are interconnected at alternating end portions 1119a and 1119b. As is seen in Figure 12, the interconnecting end portions 1119b of one segment are positioned opposite interconnecting end portions 1119a of adjacent segments. The end portions as shown are generally elliptical but may be rounded or square or pointed or the like. Any configuration of end portions is acceptable so long as it provides an undulating pattern, as shown. When the flat form 1110 is formed into an unexpanded tube as shown in Figure 13, the segments are cylindrical but the end portions 1119 of adjacent segments remain in an opposed position relative to each other.

A more preferred method of manufacture begins with a thin walled tube which is then laser cut to provide the desired configuration. It may also be chemically etched or EDM'd (electrical discharge machined) to form an appropriate configuration.

Interconnecting elements 1120 extend from one end portion 1119 of one segment 1116 to another end portion 1119 of another adjacent segment 1116 but not to an oppositely positioned end portion 1119 of an adjacent segment 1116. There are at least three struts included between the points on each side of a segment 1116 at which an interconnecting element 1120 contacts an end portion 1119. This results in the interconnecting elements 1120 extending in an angular direction between segments around the periphery of the tubular stent. Interconnecting elements 1120 are preferably of the same length but may vary from one segment to the other. Also, the diagonal direction may reverse from one segment to another extending upwardly in one case and downwardly in another, although all connecting elements between any pair of segments are substantially parallel. Figure 12, for example shows them extending downwardly, right to left. Upwardly would extend up left to right in this configuration.

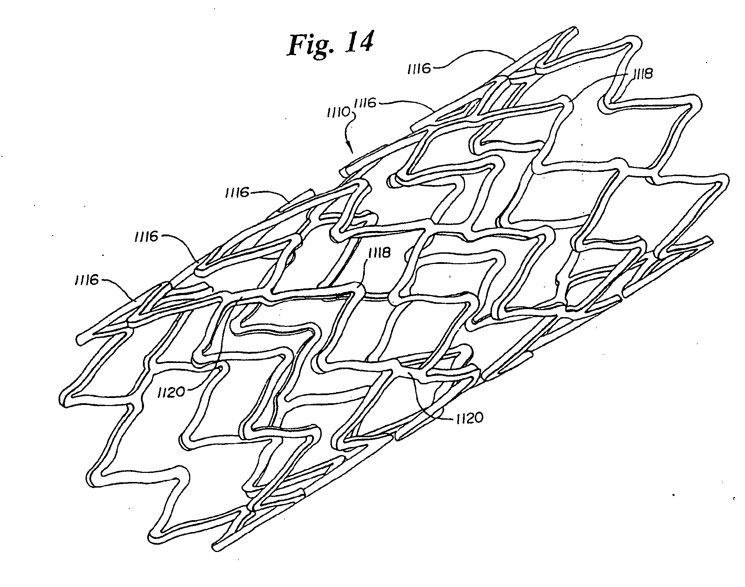

As a result of this angular extension of the interconnecting elements 1120 between adjacent segments and loops, upon expansion of the stent as seen in Figure 14, the closest adjacent end portions 1119 between segments 1116 are displaced from each other and are no longer opposite each other so as to minimize the possibility of binding or overlapping between segments, i.e., pinching.

The number of interconnecting elements 1120 may vary depending on circumstances in any particular instance. Three per segment are satisfactory for the configuration shown and at least three will be used typically.

The alternate design shown in Figure 15 includes longer struts 1118a in the two end segments 1116a than in the intermediate segments 1116. This allows the end segments (1116a) to have less compression resistance than the intermediate segments (1116), providing a more gradual transition from the native vessel to the support structure of the stent. Otherwise, the configuration is the same as that shown in Figure 12.

As indicated in the Figures 1-8, 10-15, the examples contemplate a variation of interconnecting element shapes ranging from rectilinear to curvilinear. The examples further contemplate embodiments in which all interconnecting elements are similarly oriented as well as embodiments in which adjacent sets of interconnecting elements extending between adjacent pairs of segments are oppositely oriented (e.g., Figs 7 and 8). The examples also contemplate the use of interconnecting elements which extend from a range of positions along the segments, ranging from various positions in the area in which paired struts are interconnected to other positions along the struts.

The examples also contemplate the possibility of interconnecting elements extending at an oblique angle relative to the longitudinal axis of the stent and connecting adjacent peaks and troughs on adjacent segments as well as peaks and troughs on adjacent segments which are separated by one or more peaks and/or troughs.

The examples also contemplate reversing the orientation of interconnecting elements as shown in Figs. 7 and 8.

Finally, there are preferably at least three interconnecting elements joining adjacent first and second segments although fewer or additional interconnecting elements are also contemplated.

As already indicated, this invention is applicable to self-expanding configurations, mechanically expandable configurations and to a wide variety of materials, including both metal and plastic and any other material capable of functioning as an expandable stent. For example, the stent may be of metal wire or ribbon such as tantalum, stainless steel or the like. It may be thin-walled. It may be of shape memory alloy such as Nitinol or the like, etc. The interconnecting elements may be formed integrally with the band-like elements (or segments) or may be bonded thereto via such methods as adhesive bonding, welding or any other known method of bonding.

The above examples and disclosure are intended to be illustrative and not exhaustive.

## Claims

1. A stent comprising interconnected band-like elements (720) having peaks (736) and troughs (740), the peaks (736) and troughs (740) circumferentially offset from one another, the band-like elements (720) consist of a repeating pattern of interconnected long, medium and short struts, a long strut connected to a medium strut, the medium strut connected to a short strut the short strut connected to another medium strut, the peaks including alternating small (740b) and large (736a) amplitude peaks and the troughs including alternating small (740b) and large (740a) amplitude troughs, **characterized in that** the large amplitude peaks and troughs are bulbous and the small amplitude peaks and troughs are pointed and adjacent bands are connected one to the other from large amplitude peak to large amplitude trough.

2. The stent of claim 1, wherein interconnected large amplitude peaks (736a) and large amplitude (740a) troughs are circumferentially offset from one another.

3. The stent of claim 1, wherein only the large amplitude peaks (736a) and large amplitude troughs (740a) have connectors extending therefrom.

4. The stent of claim 1-3, wherein the large amplitude peaks (736a) and the large amplitude troughs (740a) of a band-like element are symmetrically disposed about the center of the band-like element (720).

5. The stent of claim 1, wherein the large amplitude peaks (736a) within a band-like element (720) are longitudinally aligned with one another, the small amplitude peaks (740b) within a band-like element (720) are longitudinally aligned with one another, the large amplitude troughs (736a) within a band-like element (720) are longitudinally aligned with one another and the small amplitude troughs (740b) within a band-like element (720) are longitudinally aligned with one another.

## Patentansprüche

1. Stent, umfassend miteinander verbundene Band-ähnliche Elemente (720), die Spitzen (736) und Täler (740) aufweisen, wobei die Spitzen (736) und Täler (740) um den Umfang von einander versetzt sind, die Band-ähnlichen Elemente (720) aus einem sich wiederholenden Muster miteinander verbundener langer, mittlerer und kurzer Streben bestehen, wobei eine lange Strebe mit einer mittleren Strebe verbunden ist, die mittlere Strebe mit einer kurzen Strebe verbunden ist, die kurze Strebe mit einer anderen mittleren Strebe verbunden ist, die Spitzen sich abwechselnde kleine (740b) und große (736a) Amplitudenspitzen umfassen und die Täler sich abwechselnde kleine (740b) und große (740a) Amplitudentäler umfassen, **dadurch gekennzeichnet, dass** die großen Amplitudenspitzen und -täler bauchig und die kleinen Amplitudenspitzen und -täler spitz sind und benachbarte Bänder miteinander von einer großen Amplitudenspitze zu einem großen Amplitudental verbunden sind.

2. Stent nach Anspruch 1, bei dem miteinander verbundene große Amplitudenspitzen (736a) und große Amplitudentäler (740a) um den Umfang von einander versetzt sind.

3. Stent nach Anspruch 1, bei dem nur die großen Amplitudenspitzen (736a) und große Amplitudentäler (740a) Verbindungsstücke aufweisen, die sich von ihnen erstrecken.

4. Stent nach Anspruch 1 - 3, bei dem die großen Amplitudenspitzen (736a) und die großen Amplitudentäler (740a) eines Band-ähnlichen Elements symmetrisch um die Mitte des Band-ähnlichen Elements (720) angeordnet sind.

5. Stent nach Anspruch 1, bei dem die großen Amplitudenspitzen (736a) innerhalb eines Band-ähnlichen Elements (720) in Längsrichtung miteinander ausgerichtet sind, die kleinen Amplitudenspitzen (740b) innerhalb eines Band-ähnlichen Elements (720) in Längsrichtung miteinander ausgerichtet sind, die großen Amplitudentäler (736a) innerhalb eines Band-ähnlichen Elements (720) in Längsrichtung miteinander ausgerichtet sind und die kleinen Amplitudentäler (740b) innerhalb eines Band-ähnlichen Elements (720) in Längsrichtung miteinander ausgerichtet sind.

## Revendications

1. Stent comprenant des éléments de type bande interconnectés (720) ayant des pics (736) et des creux (740), les pics (736) et les creux (740) étant décalés de manière périphérique les uns des autres, les éléments de type bande (720) étant constitués d'un motif répétitif d'entretoises longues, moyennes et courtes interconnectées, une entretoise longue étant connectée à une entretoise moyenne, l'entretoise moyenne étant connectée à une entretoise courte, l'entretoise courte étant connectée à une autre entretoise moyenne, les pics comprenant des pics de faible amplitude (740b) et de grande amplitude (736a) en alternance et les creux comprenant des creux de faible amplitude (740b) et de grande amplitude (740a) en alternance, **caractérisé en ce que** les pics et creux de grande amplitude sont en forme de boule et les pics et creux de faible amplitude sont pointus et des bandes adjacentes sont connectées l'une à l'autre d'un pic de grande amplitude à un creux de grande amplitude.

2. Stent selon la revendication 1, dans lequel des pics de grande amplitude (736a) et des creux de grande amplitude (740a) interconnectés sont décalés de manière périphérique les uns des autres.

3. Stent selon la revendication 1, dans lequel seuls les pics de grande amplitude (736a) et les creux de grande amplitude (740a) ont des connecteurs s'étendant à partir d'eux.

4. Stent selon les revendications 1 à 3, dans lequel les pics de grande amplitude (736a) et les creux de grande amplitude (740a) d'un élément de type bande sont disposés symétriquement autour du centre de l'élément de type bande (720).

5. Stent selon la revendication 1, dans lequel les pics de grande amplitude (736a) au sein d'un élément de type bande (720) sont alignés longitudinalement les uns avec les autres, les pics de faible amplitude (740b) au sein d'un élément de type bande (720) sont alignés longitudinalement les uns avec les autres, les creux de grande amplitude (736a) au sein d'un élément de type bande (720) sont alignés longitudinalement les uns avec les autres et les creux de faible amplitude (740b) au sein d'un élément de type bande (720) sont alignés longitudinalement les uns avec les autres.
